# FASCICULE DE BREVET EUROPEEN

(11) **EP 1 194 087 B1**
(45) Date de publication et mention de la délivrance du brevet: **27.08.2008**
(21) Numéro de dépôt: 00949674.6
(22) Date de dépôt: 07.07.2000
(51) Int. Cl.: A61F 2/44, A61F 2/46

(54) **IMPLANT INTERSOMATIQUE ANATOMIQUE**
ANATOMISCHES ZWISCHENKÖRPER-IMPLANTAT
ANATOMICAL INTERBODY IMPLANT

(30) Priorité: 09.07.1999 FR 9909122
(43) Date de publication de la demande: 10.04.2002
(73) Titulaire: SCIENT'X, 75007 Paris (FR)
(72) Inventeur: BERNARD, Pierre, F-33000 Bordeaux (FR); POINTILLART, Vincent, F-33000 Bordeaux (FR)
(74) Mandataire: Thibault, Jean-Marc
(86) Numéro de dépôt international: PCT/FR2000/001967
(87) Numéro de publication internationale: WO 2001/003615

(56) Documents cités:
- WO-A-00/25706
- WO-A-98/48738
- DE-U- 29 916 078
- FR-A- 2 703 580
- FR-A- 2 727 003
- FR-A- 2 747 034
- FR-A- 2 782 914
- US-A- 5 683 464
- US-A- 5 865 845

## Description

### DOMAINE TECHNIQUE :

La présente invention concerne un implant intersomatique destiné à être inséré dans l'espace discal défini entre deux vertèbres adjacentes, en vue de restituer une hauteur convenable intervertébrale et d'assurer une fusion osseuse entre lesdites vertèbres adjacentes.

L'objet de l'invention vise, plus précisément, un implant intersomatique du type cervical, destiné à être logé dans l'espace discal défini entre deux vertèbres cervicales adjacentes.

### TECHNIQUE ANTERIEURE :

Dans l'état de la technique, il est connu d'insérer un implant intersomatique dans l'espace discal défini entre deux vertèbres cervicales adjacentes. De nombreuses formes de réalisation de tels implants intersomatiques ont été proposées dans l'art antérieur. Par exemple, il est connu par le document FR 2 703 580 un implant intersomatique. destiné à être inséré dans l'espace discal défini entre deux vertèbres voisines en vue du rétablissement anatomique de l'espace intervertébral, l'implant se présentant sous la forme d'une cage de forme générale parallélépipédique comportant au moins deux parois sagittales sensiblement parallèles à un plan sagittal et reliées entre elles par au moins des parois transversales antérieure et postérieure sensiblement parallèles à un plan frontal. Les parois délimitent entre elles un volume ouvert pour un remplissage osseux et présentent des rebords s'étendant d'un coté pour délimiter une première face transversale et de l'autre côté pour délimiter une deuxième face transversale. Le profil de chaque face transversale est délimité par des protubérances aménagées sur les rebords des parois sagittales et frontales.

Pour respecter au mieux l'anatomie de la colonne vertébrale, le document US-A 683 464 décrit un implant intersomatique comportant des faces transversales qui peuvent avoir des profils convexes dans le plan sagittal et/ou dans le plan frontal. De même, le document US-A-5 865 845 propose un implant intervertébral où les profils des deux faces transversales sont convexes et congruents aux profils des vertèbres sus et sous-jacentes dans le plan sagittal. Le profil des faces transversales de cet implant dans le plan frontal est concave ou plan.

D'une manière générale, il doit être considéré que l'insertion d'un implant du type décrit dans l'art antérieur, dans l'espace discal de deux vertèbres adjacentes est susceptible de conduire à un positionnement incorrect des vertèbres entre elles. Il s'ensuit qu'il ne peut pas être obtenu une bonne reconstitution osseuse entre les vertèbres concernées.

L'objet de l'invention vise donc à remédier aux inconvénients énoncés ci-dessus en proposant un implant intersomatique adapté pour respecter au mieux l'anatomie de la colonne vertébrale.

### EXPLOSE DE L'INVENTION :

Pour atteindre un tel objectif, l'implant intersomatique selon l'invention est conforme à la revendication 1

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

### BREVE DESCRIPTION DES DESSINS :

La **fig. 1** est une vue en perspective d'un exemple de réalisation d'un implant conforme à l'invention.
La **fig. 2** est une vue frontale d'un implant prise sensiblement selon la flèche **f₂** de la **fig. 1****.**
La **fig. 3** est une vue sagittale d'un implant prise sensiblement selon la flèche **f₃** de la **fig. 1****.**
La **fig. 4** est une vue de dessus d'une pince de préhension d'un implant conforme à l'invention.
La **fig.** 5 est une vue en perspective montrant un implant intersomatique supporté par une pince de préhension.

### MEILLEURE MANIERE DE REALISER L'INVENTION :

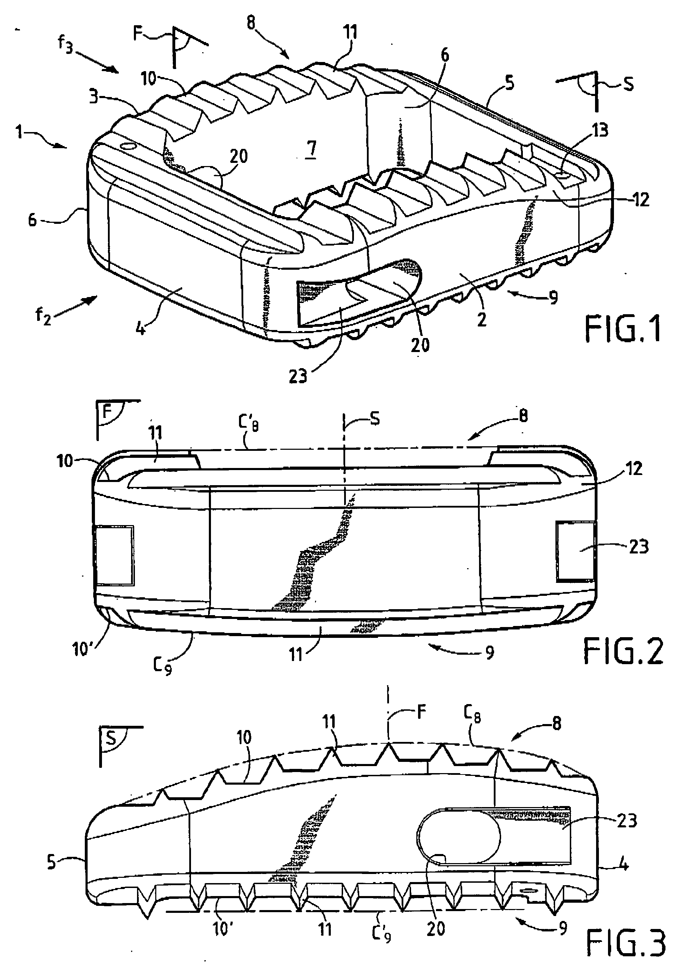

Tel que cela apparaît plus précisément aux **fig. 1** à **3**, l'implant intersomatique conforme à l'invention se présente sous la forme d'une cage **1** présentant une forme générale parallélèpipédique et destinée à être insérée dans l'espace discal entre deux vertèbres adjacentes, par exemple cervicales. La cage **1** comporte une première paroi sagittale **2** et une seconde paroi sagittale **3** s'étendant sensiblement parallèlement l'une à l'autre et à un plan S dit sagittal ou antéro-postérieur. Les parois sagittales **2** et **3** sont reliées entre elles par une paroi transversale, dite antérieure **4,** et par une paroi transversale, dite postérieure **5** s'étendant parallèlement l'une à l'autre et à un plan frontal F perpendiculaire au plan sagittal S.

Il est à noter que la cage **1** peut comporter une ou plusieurs parois médianes ou intermédiaires s'étendant sensiblement parallèlement aux parois transversales et/ou sagittales. De préférence, des congés de raccordement **6** sont aménagés entre les parois sagittales et les parois transversales, d'une part, selon leurs faces verticales internes et, d'autre part, selon leurs faces verticales externes, permettant de disposer d'une cage **1** avec des coins arrondis sur ses faces verticales externes et internes. Par exemple, les parois **2** à **5** présentent une épaisseur sensiblement de même valeur. De même, la hauteur de la paroi transversale antérieure **4** présente une valeur supérieure à la hauteur de la paroi transversale postérieure **5 (****fig. 3**).

La cage **1** présente intérieurement un volume **7** délimité par les faces verticales internes des parois **2** à **5** et destiné à être rempli par un produit de comblement osseux destiné à la fusion intersomatique. Ce volume 7 s'ouvre selon une première face transversale **8,** dite supérieure dans l'exemple illustré, et une deuxième face transversale **9,** dite inférieure dans l'exemple considéré. Les parois **2** à **5** présentent, d'un côté, des rebords **10** délimitant la face transversale supérieure **8** et, de l'autre côté, des rebords **10'** délimitant la face transversale inférieure **9.**

La cage **1** comporte des protubérances ou des saillies **11** aménagées sur les rebords **10, 10'** des parois **2** à **5** pour permettre un accrochage de la cage sur les vertèbres sus et sous-jacentes. Les protubérances **11** sont constituées dans l'exemple préféré illustré, par des crans s'étendant parallèlement les uns des autres et par rapport au plan frontal **F.** Bien entendu, les protubérances peuvent présenter des formes différentes et être réalisées, par exemple, par des picots individuels ou par des crans en forme de chevrons. D'une manière générale, il doit être compris que les faces transversales supérieure **8** et inférieure **9** correspondent à l'enveloppe passant par le sommet des protubérances **11.**

Selon une caractéristique de l'invention qui est illustrée plus précisément à la **fig. 3****,** la face transversale supérieure **8** présente dans le plan sagittal S, un profil convexe **C₈** congruent ou complémentaire du profil anatomique sagittal d'une vertèbre voisine ou sus-jacente dans l'exemple illustré. Il doit être compris que les rebords **10** des parois et, plus précisément, les protubérances **11** délimitant cette face transversale supérieure **8,** sont aménagés pour s'inscrire dans une enveloppe dont la section dans le plan sagittal S, est de forme bombée ou convexe.

Selon une autre caractéristique préférée de réalisation, la face transversale supérieure **8** est délimitée dans le plan frontal **F** par un profil rectiligne ou droit **C'₈** (**fig. 2**). De préférence, les rebords **10** des parois **2** à **5** délimitant la face transversale supérieure **8** sont aménagés pour être raccordés aux faces extérieures des parois **2** à **5** par des congés de raccordement **12.**

Selon une autre caractéristique de l'invention qui apparaît plus précisément à la **fig. 2****,** la face transversale inférieure **9** présente, dans le plan frontal **F,** un profil convexe **C₉** congruent ou complémentaire au profil anatomique frontal d'une vertèbre voisine ou sous-jacente dans l'exemple illustré. Les rebords **10'** des parois **2** à **5** et, plus précisément, les protubérances **11** délimitant cette face transversale **9** sont aménagées pour s'inscrire dans une enveloppe dont la section dans le plan S, est de forme bombée.

Par ailleurs, il est à noter que la face transversale inférieure **9** présente, dans le plan sagittal, un profil **C'₉** qui est sensiblement droit.

Avantageusement, la cage **1** décrite ci-dessus est adaptée pour recevoir au moins un et, dans l'exemple illustré, deux repères radio-opaques **13** incorporés sur au moins une partie de la hauteur de la cage au niveau des parois transversales antérieure **4** et postérieure 5.

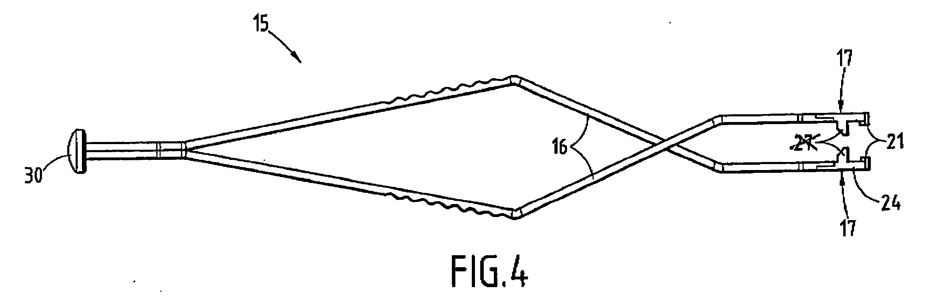

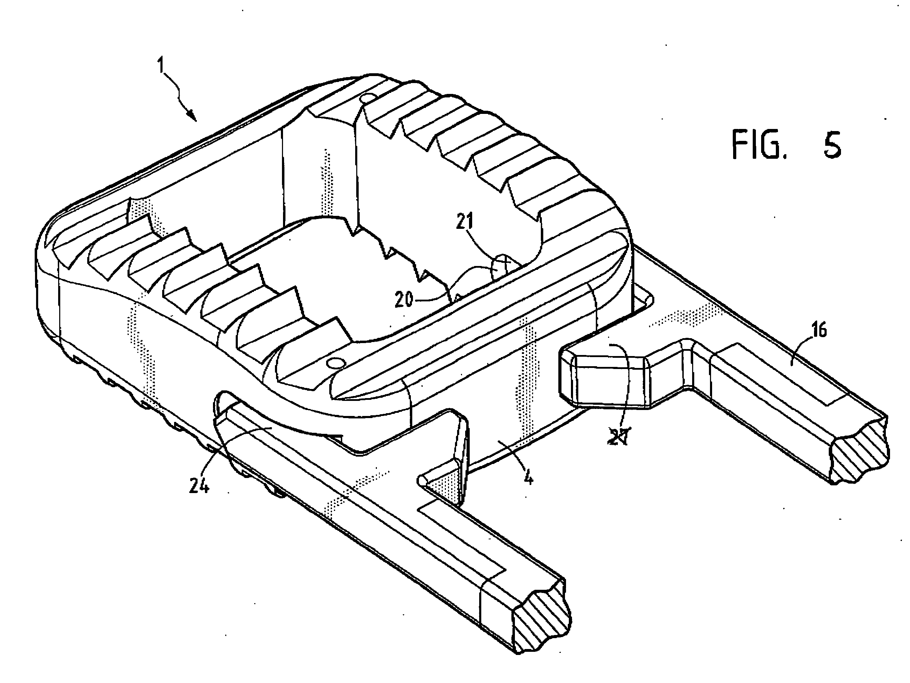

La cage **1** décrite ci-dessus est particulièrement adaptée pour permettre sa manipulation par une pince de préhension **15,** telle qu'illustrée aux **fig. 4** et 5, comportant deux branches **16** munies chacune, à chaque extrémité, d'un mors de préhension **17.**

A cet effet, la cage **1** comporte deux logements **20** s'étendant dans le prolongement l'un de l'autre et adaptés pour recevoir chacun un téton radial **21** aménagé sur chaque mors **17** de la pince. Dans l'exemple illustré, les logements **20** sont réalisés sur les parois sagittales **2** et **3** en étant alignés et en s'étendant selon une direction frontale perpendiculaire au plan sagittal **S.** Les logements **20** sont aménagés, de préférence, à proximité de la paroi transversale antérieure **4.** Dans l'exemple illustré, chaque logement **20** débouche sur les deux faces verticales opposées des parois **2** et **3.** Bien entendu, il peut être prévu de réaliser les logements **20** au niveau de la paroi transversale antérieure **4** en s'étendant selon une direction frontale perpendiculaire au plan sagittal S. Dans cette forme de réalisation, il est à noter que les deux logements **20** peuvent comnuniquer entre-eux directement pour constituer un alésage unique. Les logements **20** possèdent une section droite transversale adaptée pour recevoir un téton radial **21** et, par exemple, sensiblement elliptique dans l'exemple illustré.

Selon une caractéristique préférée de réalisation, la cage **1** comporte des moyens **23** d'anti-rotation destinés à coopérer avec des moyens complémentaires **24** aménagés sur les mors **17** de la pince de préhension, de manière à obtenir, en position de préhension de la cage par la pince, un blocage relatif en rotation entre la cage **1** et la pince **15.** Dans l'exemple illustré, ces moyens d'anti-rotation **23** sont constitués par une rainure aménagée dans chaque paroi sagittale **2, 3** pour s'ouvrir dans un logement **20** correspondant et en s'étendant jusqu'à la face externe de la paroi transversale antérieure **4.** Comme illustré plus particulièrement à la **fig. 3****,** chaque rainure **23** possède une section droite transversale rectangulaire.

Tel que cela apparaît plus précisément aux **fig. 4** et 5, chaque mors de préhension **17** est aménagé pour présenter, en tant que moyen d'anti-rotation complémentaire **24,** un bras ou un barreau pourvu, à son extrémité libre, d'un téton radial **21** s'étendant en alignement l'un de l'autre. Chaque bras **24,** qui présente une section transversale complémentaire de la rainure **23,** est destiné à être engagé au moins en partie, dans la rainure **23** aménagée sur une paroi sagittale lorsque chaque téton **21** est engagé dans un logement complémentaire **20.**

L'engagement des tétons **21** dans les logements **20** assure la préhension de la cage et son blocage en translation, tandis que la coopération des bras **24** avec les rainures **23** permet d'obtenir un blocage en rotation, notamment selon une direction frontale. Il s'ensuit un blocage complet de la cage par les mors de préhension **17.** Il est à noter qu'il pourrait être envisagé de réaliser de manière différente, les moyens d'antirotation **23, 24.** Par exemple, il pourrait être prévu de réaliser des logements **20** de forme prismatique destinés à coopérer avec des tétons de forme complémentaire.

### POSSIBILITE D'APPLICATION INDUSTRIELLE :

La cage **1** décrite ci-dessus est particulièrement adaptée pour respecter la forme de l'espace discal défini entre deux vertèbres, par exemple cervicales. Le respect de l'anatomie du disque intervertébral remplacé par la cage **1** favorise la fusion osseuse entre les vertèbres et la restauration de la statique rachidienne. De plus, la mise en place de la cage **1** est particulièrement simplifiée par l'utilisation de la pince de préhension **15**. Ainsi, à partir d'une voie d'abord antérieure du rachis cervical, il est procédé à une résection des ostéophytes, à une dissectomie, puis à un avivement des plateaux des vertèbres. Ensuite, une cage **1** peut être prise par la pince **15** en assurant, par une action sur les branches **16** pour écarter les mors **17,** le positionnement relatif entre les tétons **21** et les logements **20,** et par une action sur les branches pour rapprocher les mors **17,** l'engagement, d'une part, des tétons **21** dans les logements **20** et, d'autre part, des bras **24** dans les rainures **23.** Il est à noter que les rainures **23** sont à même d'assurer une fonction de guidage pour les tétons **21** qui se trouvent amenés jusqu'au logement, afin de s'y introduire. Dans cette position, la cage **1** est parfaitement bloquée par rapport à la pince, grâce à l'engagement des tétons **21** dans les logements **20** et des bras **24** dans les rainures **23, 4.** La cage **1** peut être introduite dans l'espace discal avec éventuellement l'application d'efforts de poussée sur l'extrémité **30** de la pince. Une action sur les branches **16** pour écarter les mors **17** permet d'assurer le dégagement des tétons **21** des logements **20,** en vue du retrait de la pince.

L'invention n'est pas limitée aux exemples décrits et représentés, car diverses modifications peuvent y être apportées sans sortir de son cadre.

## Revendications

1. Implant intersomatique destiné à être inséré dans l'espace discal défini entre deux vertèbres voisines, dites sus et sous-jacentes, en vue du rétablissement anatomique de l'espace intervertébral, l'implant se présentant sous la forme d'une cage (**1**) de forme générale parallélépipédique comportant au moins deux parois sagittales (**2**, **3**) sensiblement parallèles à un plan sagittal (**S**) et reliées entre elles par au moins des parois transversales antérieure (**4**) et postérieure (**5**) sensiblement parallèles à un plan frontal (**F**), les parois (**2** à **5**) délimitant entre elles un volume ouvert (**7**) pour un remplissage osseux et présentant des rebords (**10**, **10'**) s'étendant d'un côté pour délimiter une première face transversale (8) et de l'autre côté, pour délimiter une deuxième face transversale (**9**), le profil (C₈, C₉) de chaque face transversale **(8, 9**) étant délimité par des protubérances (**11**) aménagées sur les rebords (**10**, **10')** des parois sagittales et frontales,
**caractérisé en ce que :**
- la première face transversale (**8**) présente dans le plan sagittal, un profil convexe (**C₈**) destiné à être congruent au profil anatomique sagittal d'une vertèbre sus-jacente,
- la deuxième face transversale présente dans le plan frontal, un profil convexe (**C₉**) destiné à être congruent au profil anatomique frontal d'une vertèbre sous-jacente.

2. Implant selon la revendication 1, **caractérisé en ce que** les rebords (**10**, **10')** des parois sagittales et frontales comportent des protubérances (**11**) formant des crans s'étendant parallèlement les uns des autres et par rapport au plan frontal (**F**).

3. Implant selon la revendication 1 ou 2, **caractérisé en ce qu'**il comporte au moins un repère radio opaque (**13**) s'étendant au moins sur une partie de la hauteur d'une paroi.

4. Implant selon l'une des revendications 1 à 3, **caractérisé en ce qu'**il comporte deux logements de réception (**20**) pour les mors (**17**) d'une pince de préhension, les logements s'étendant sensiblement en vis-à-vis selon une direction frontale perpendiculaire au plan sagittal (**S**) de la cage.

5. Implant selon la revendication 4, **caractérisé en ce que** chaque logement (**20**) s'ouvre au moins sur la face externe d'une paroi sagittale (**2**, **3**).

6. Implant selon la revendication 4 ou 5, **caractérisé en ce que** les parois sont aménagées pour comporter des moyens d'anti-rotation (**23**) destinés à coopérer avec des moyens complémentaires (**24**) aménagés sur les mors (**17**) de la pince de préhension, de manière à obtenir, en position de préhension de la cage par la pince, un blocage relatif entre la cage et la pince.

7. Implant selon la revendication 6, **caractérisé en ce que** chaque logement (**20**) s'ouvre sur les parois sagittales (**2**, **3**) dans une rainure (23) s'étendant jusqu'à la face externe de la paroi antérieure pour constituer les moyens d'anti-rotation et permettre l'insertion des mors d'une pince de préhension.

## Claims

1. An intersomatic implant designed to be inserted in the disk space defined between two adjacent vertebrae, namely an overlying vertebra and an underlying vertebra, for the purpose of reestablishing the anatomic space between the vertebrae, the implant being in the form of a cage (1) that is generally in the shape of a rectangular block having at least two sagittal walls (2, 3) substantially parallel to a sagittal plane (S) and interconnected at least by an anterior transverse wall (4) and by a posterior transverse wall (5) extending substantially parallel to a frontal plane (F), the walls (2 to 5) defining between them an open volume (7) for bone filler and presenting rims (10, 10') extending on one surface to define a first transverse face (8) and on the opposite surface to define a second transverse face (9), the profile (C₈, C₉) of each transverse face (8, 9) being defined by protuberances (11) formed on the rims (10, 10') of the sagittal and frontal walls,
the implant being **characterised in that:**
- the first transverse face (8) presents in the sagittal plane a convex profile (C₈) designed to be congruent with the sagittal anatomic profile of an overlying vertebra; and
- the second transverse face presents in the frontal plane a convex profile (C₉) designed to be congruent with the frontal anatomic profile of an overlying vertebra.

2. An implant according to claim 1, **characterised in that** the rims (10, 10') of the sagittal and frontal walls carry protuberances (11) forming ridges extending parallel to one another and to the frontal plane (F).

3. An implant according to claim 1 or claim 2, **characterised in that** it has at least one radio-opaque marker (13) extending over at least a portion of the height of a wall.

4. An implant according to one of claims 1 to 3, **characterised in that** it has two housings (20) for receiving the jaws (17) of a manipulation forceps, the housings extending substantially facing each other in a frontal direction perpendicular to the sagittal plane (S) of the cage.

5. An implant according to claim 4, **characterised in that** each housing (20) opens out at least to the external face of one of the sagittal walls (2, 3).

6. An implant according to claim 4 or claim 5, **characterised in that** the walls are arranged to include antirotation means (23) for co-operating with complementary means (24) arranged on the jaws (17) of the manipulation forceps so that, when the cage is engaged by the forceps, the cage is prevented from moving relative to the forceps.

7. An implant according to claim 6, **characterised in that** each housing (20) opens to the sagittal walls (2, 3) in a respective groove (23) extending to the external face of the anterior wall so as to constitute the antirotation means and so as to enable the jaws of a manipulation forceps to be inserted.

## Patentansprüche

1. Zwischenkörperimplantat, das dafür bestimmt ist, mit dem Ziel der anatomischen Wiederherstellung des Zwischenwirbelraums in den Bandscheibenraum eingesetzt zu werden, der zwischen zwei benachbarten Wirbeln, dem darüberliegenden und darunterliegenden, definiert ist, wobei das Implantat in Form eines Käfigs (1) mit einer im allgemeinen parallelepipedischen Form vorliegt, der mindestens zwei sagittale Wände (2, 3) aufweist, die im wesentlichen parallel zu einer sagittalen Ebene (S) verlaufen und untereinander über mindestens die vordere (4) und hintere (5) Querwand verbunden sind, die im wesentlichen parallel zu einer Frontalebene (F) verlaufen, wobei die Wände (2 bis 5) zwischen sich ein offenes Volumen (7) für eine Knochenfüllung begrenzen und Ränder (10, 10') aufweisen, die sich einerseits erstrecken, um eine erste Querseite (8) zu begrenzen und andererseits, um eine zweite Querseite (9) zu begrenzen, wobei das Profil (C₈, C₉) jeder Querseite (8, 9) von den Vorsprüngen (11) begrenzt wird, die auf den Rändern (10, 10') der sagittalen und frontalen Wände angeordnet sind,
**dadurch gekennzeichnet, daß:**
- die erste Querseite (8) in der sagittalen Ebene ein konvexes Profil (C₈) aufweist, das dafür bestimmt ist, mit dem sagittalen anatomischen Profil eines darüberliegenden Wirbels übereinzustimmen,
- die zweite Querseite in der Frontalebene ein konvexes Profil (C₉) aufweist, das dafür bestimmt ist, mit dem frontalen anatomischen Profil eines darunterliegenden Wirbels übereinzustimmen.

2. Implantat nach Anspruch 1, **dadurch gekennzeichnet, daß** die Ränder (10, 10') der sagittalen und frontalen Wände Vorsprünge (11) aufweisen, die Stufen bilden, die parallel zueinander und in Bezug auf die Frontalebene (F) verlaufen.

3. Implantat nach Anspruch 1 oder 2, **dadurch gekennzeichnet, daß** es mindestens eine röntgenstrahlenundurchlässige Markierung (13) aufweist, die sich mindestens über einen Teil der Höhe einer Wand erstreckt.

4. Implantat nach einem der Ansprüche 1 bis 3, **dadurch gekennzeichnet, daß** es zwei Aufnahmen (20) für die Backen (17) einer Greifzange aufweist, wobei sich die Aufnahmen im wesentlichen einander gegenüberliegend in einer frontalen Richtung senkrecht zur sagittalen Ebene (S) des Käfigs erstrecken.

5. Implantat nach Anspruch 4, **dadurch gekennzeichnet, daß** sich jede Aufnahme (20) mindestens zur Außenseite einer sagittalen Wand (2, 3) hin öffnet.

6. Implantat nach Anspruch 4 oder 5, **dadurch gekennzeichnet, daß** die Wände eingerichtet sind, um Verdrehsicherungsmittel (23) aufzuweisen, die dafür bestimmt sind, mit ergänzenden Mitteln (24) zusammenzuwirken, die an den Backen (17) der Greifzange angeordnet sind, um in der Position, in der die Zange den Käfig greift, eine relative Blockade zwischen Käfig und Zange zu erreichen.

7. Implantat nach Anspruch 6, **dadurch gekennzeichnet, daß** sich jede Aufnahme (20) zu den sagittalen Wänden (2, 3) hin in einer Nut (23) öffnet, die sich bis zur Außenseite der vorderen Wand erstreckt, um die Verdrehsicherungsmittel zu bilden und das Einsetzen der Backen einer Greifzange zu ermöglichen.
